# EUROPEAN PATENT SPECIFICATION

(11) **EP 3 407 933 B1**
(45) Date of publication and mention of the grant of the patent: **16.06.2021**
(21) Application number: 17702828.9
(22) Date of filing: 27.01.2017
(51) Int. Cl.: A61M 1/36, A61M 5/168

(54) **SOLUTION INFUSION APPARATUS, PARTICULARLY FOR DIALYSIS SYSTEMS**
LÖSUNGSINFUSIONSVORRICHTUNG, INSBESONDERE FÜR DIALYSESYSTEME
APPAREIL DE PERFUSION DE SOLUTION, PARTICULIÈREMENT POUR SYSTÈMES DE DIALYSE

(30) Priority: 27.01.2016 IT UB20160390
(43) Date of publication of application: 05.12.2018
(73) Proprietor: Bellco S.r.l., 41037 Mirandola (IT)
(72) Inventor: PIRAZZOLI, Paolo, 41030 San Prospero (IT); PEDRAZZI, Renato, 41037 Mirandola (IT)
(74) Representative: Maschio, Antonio
(86) International application number: PCT/EP2017/051813
(87) International publication number: WO 2017/129770

(56) References cited:
- EP-A1- 2 641 625
- US-A- 5 200 090

## Description

The present invention relates to a solution infusion apparatus, particularly for an anticoagulant solution, particularly for dialysis systems.

Currently, in a dialysis machine designed for acute treatments, anticoagulation normally occurs by infusing into the blood circuit of the machine an anticoagulant solution. For example, a citrate solution can be provided to blood using a suction pump which draws the citrate solution from one or more pouches. The suction pump can be a peristaltic pump or a positive-displacement perfusion pump.

The pouches are normally hung from a balance which provides the control system of the suction pump with information on the weight of the infused liquid. When the pouches are empty, the pump is deactivated and an alarm signal is activated, warning the operator of the need to replace the empty pouches with new pouches filled with anticoagulant solution. The operator then replaces the empty pouches and manually reactivates the suction pump.

This normal type of procedure with the apparatus discussed for infusing anticoagulant solutions, however, is not free from drawbacks. The first drawback is that during the replacement of the pouch, the suction pump is motionless and the blood circuit operates without infusion of anticoagulant. When the pump is motionless, this can cause coagulation in the blood circuit, with a consequent increase in the pressures present in the circuit and the need to interrupt the treatment, often without having the possibility to continue to return the blood to the patient.

Operators often prefer to replace pouches that are about to empty before this occurs, i.e., before this is detected by the balance and therefore before the suction pump is deactivated. In this case, if the operator does not remember to interrupt the operation of the pump manually, during the period between detection by the balance of the lack of the pouch, the activation of the corresponding alarm signals and the interruption of the suction pump, the pump itself continues pumping without available pouch solution and draws air through the connector that connected it hydraulically to the pouch.

If the procedure for replacing an empty pouch is not performed correctly, e.g.. the operator forgets to interrupt the operation of the pump before removing the pouch, the apparatus can enter an alarm mode. Additionally the pump may be stopped by (1) inappropriately removing the empty pouch, (2) abnormally changing the balance readings, or (3) the operator adds a full pouch out of sequence. In all these cases, due to the abnormal reading by the balance, the operation of the pump can be interrupted and then must be restored.

Upon restarting the pump, the air aspirated by the pump prior to the activation of the alarm signal is introduced in the blood circuit, where it can cause the forming of foam, the malfunction of the dialysis machine, and the consequent activation of the alarm signals, and other problems that are difficult to manage by the operator which often entails the need to interrupt patient treatment.

A further drawback of these infusion apparatuses of the known type resides in that the alarms for replacing the pouches are activated when there is still a certain residual quantity of anticoagulant solution left in the pouches. The residual amounts left in the pouches is done to give time to the operator to finish what he/she is doing before focusing on pouch replacement. Providing early alarms, however, generally results in wasted anticoagulant solution.

US5200090A describes an example of a system for weighing and monitoring flow from multiple fluid sources into a flow system wherein incoming fluid is receiving in a weighing bag attached to a control system for monitoring the amount of fluid passed through the weighing bag and for preventing admission of air into the flow system. An alarm attached to the weighing bag warns when the fluid in the weighing bag is approaching empty, and the system automatically shuts down if the fluid in the weighing bag is not replenished.

EP2641625A1 describes an example of extracorporeal blood treatment apparatus with multiple treatment solution reservoirs. Weight signals are transmitted to a control unit that is capable of monitoring the weight changes of a reservoir and to control a pump acting on an exit line from said reservoir.

The aim of the present invention is to provide a solution infusion apparatus that solves the technical problems described above, obviates the drawbacks and overcomes the limitations of the background art, with assurance to the patient of a continuous infusion of this solution, without interruptions of any kind.

Within this aim, an object of the present invention is to provide an infusion apparatus that fully prevents the introduction of air in the blood circuit of the dialysis machine.

Another object of the invention is to provide an infusion apparatus that can operate correctly even in case of human errors made by the operators.

A further object of the invention is to provide an infusion apparatus that allows to use completely, without interruptions and in a very simple manner, all of the solution contained in the pouches.

Another object of the invention is to provide an infusion apparatus in which pouch replacement is fast and simple to perform.

A further object of the invention is to provide an infusion apparatus that is capable of giving the greatest assurances of reliability and safety in use.

Another object of the invention is to provide an infusion apparatus that is easy to provide and economically competitive if compared with the background art.

This aim, these objects and others that will become better apparent are achieved by a solution infusion apparatus, particularly for dialysis systems, comprising at least one main pouch adapted to contain a solution and comprising a pump configured for introducing said solution in the blood circuit of a dialysis system, characterized in that it comprises at least one additional pouch that is hydraulically connected to said at least one main pouch, said pump being configured to draw said solution from said at least one additional pouch.

Further characteristics and advantages of the present invention will become better apparent from the description of a preferred but not exclusive embodiment of a solution infusion apparatus, particularly for dialysis systems, illustrated by way of nonlimiting example with the aid of the accompanying drawings, wherein:

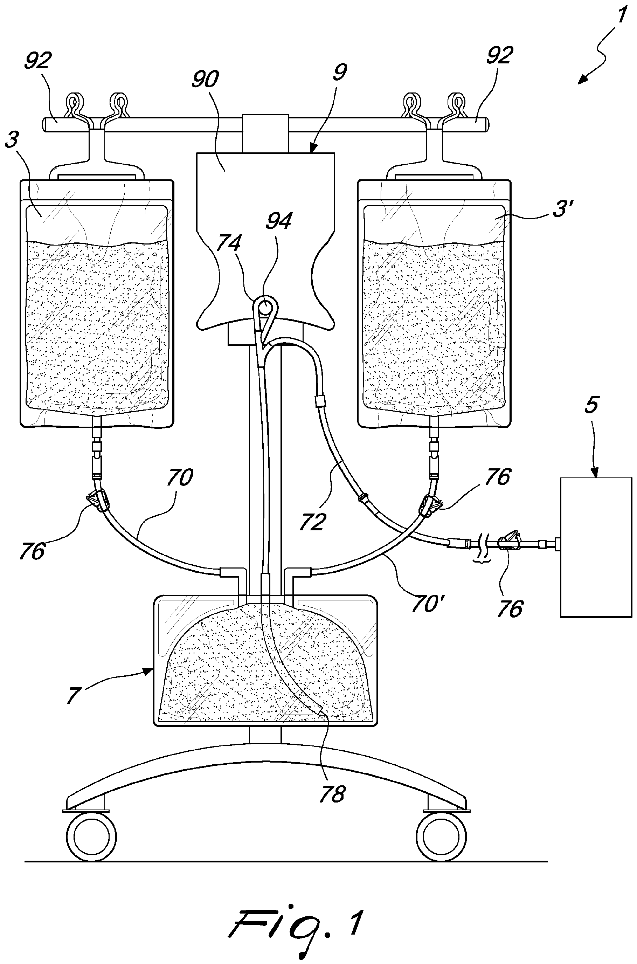

Figure 1 is a schematic front view of an embodiment of an infusion apparatus, according to the invention, shown in a first configuration for use;
Figure 2 is a schematic front view of the apparatus of Figure 1, according to the invention, shown in a second configuration for use;

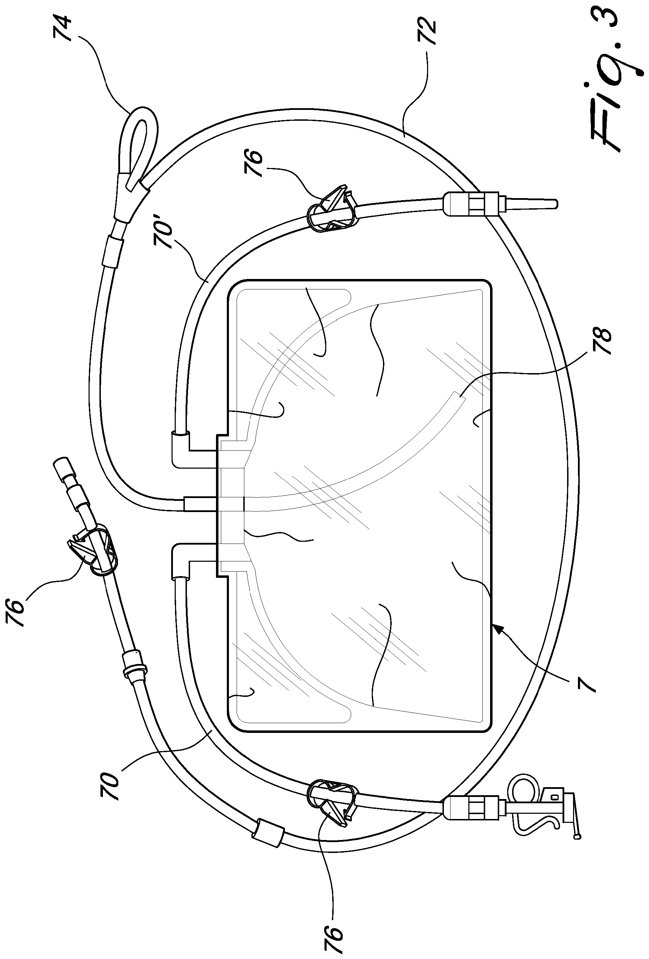

Figure 3 is a view of an additional pouch that belongs to the apparatus of Figure 1, according to the invention.

The present invention is defined in independent claims 1 and 6 and certain optional features thereof are defined in the dependent claims. Referring to the figures, a solution infusion apparatus, particularly for dialysis systems, designated by the reference numeral 1, comprises at least one main pouch 3, 3' that is adapted to contain a solution and a pump 5 configured to introduce this solution in the blood circuit of a dialysis system.

According to the invention, the infusion apparatus 1 comprises at least one additional pouch 7, which is connected hydraulically to the main pouch 3, 3', wherein the pump 5 is configured to draw this solution from the additional pouch 7.

Advantageously, as described hereinafter, this solution is an anticoagulant solution. However, this solution can also be an electrolytic infusion solution.

The anticoagulant solution contained in the additional pouch 7 and in the main pouch 3, 3' preferably comprises citrate.

The additional pouch 7 is connected hydraulically to the main pouch 3, 3', advantageously with a first hydraulic duct 70, 70'.

Advantageously, the additional pouch 7 is connected hydraulically to the pump 5 with a second hydraulic duct 72, which draws, at the end 78, the anticoagulant solution within the additional pouch 7 to introduce it in the blood circuit of the dialysis system.

The additional pouch 7 is arranged at a lower level than the main pouch 3, 3' so the anticoagulant solution contained in the main pouch 3, 3' fills by gravity the additional pouch 7.

The infusion apparatus 1 can comprise a plurality of main pouches 3 and 3', each of which is hydraulically connected to the additional pouch 7.

As in the accompanying figures, the apparatus 1 comprises a hydraulic duct 70 and 70' between each one of the main pouches 3 and 3' and the additional pouch 7.

Both main pouches 3 and 3' fill by gravity, in parallel regarding each other, the additional pouch 7.

Advantageously, the additional pouch 7 can comprise a volume of anticoagulant solution comprised in the interval between 300 milliliters and 1 liter, preferably comprised in the interval between 400 and 700 milliliters, and even more preferably comprised in the interval between 500 and 600 milliliters.

The infusion apparatus 1 comprises means 9 for detecting the total weight of the anticoagulant solution in the additional pouch 7 and in all the main pouches 3 and 3' associated with the apparatus. These weight detection means 9 can comprise a balance 90.

The main pouches 3 and 3' and the additional pouch 7 hang advantageously from the balance 90.

Advantageously, the balance 90 can comprise protruding arms 92 for supporting the main pouches 3 and 3'.

The balance 90 can further comprise a hook 94 on which the additional pouch 7 is hung.

The additional pouch 7 can comprise a supporting loop 74 which can be associated with the hook 94 to hang the pouch 7 from the balance 90. Advantageously, the supporting loop 74 can be associated directly with the second hydraulic duct 72.

The infusion apparatus 1 comprises means for signaling the emptying of the main pouch 3, 3', which are associated with the means 9 for detecting the total weight of the anticoagulant solution.

Substantially, the infusion apparatus 1 can comprise a control system capable of detecting the total weight variations of the main pouches 3 and 3' and of the additional pouch 7, to signal the imminent emptying of the main pouches 3 and 3' and optionally also of the additional pouch 7.

Advantageously, the pump 5 of the infusion apparatus 1, in the absence of all the main pouches 3 and 3', is configured to continue to perform in an open loop drawing the anticoagulant solution from the additional pouch 7.

When main pouches 3 and 3' are empty, the means 9 for detecting the total weight detect a weight that corresponds to the weight of the anticoagulant solution present in the additional pouch 7 alone. The apparatus 1 therefore signals this situation to the operator, by the signaling means, but keeps the pump 5 running, said pump continuing to introduce anticoagulant solution, drawn from the additional pouch 7, into the blood circuit of the dialysis system.

In this step, the control system operates in an open loop, ignoring the information that arrives from the means 9 for detecting the total weight. Therefore, the user can detach the main pouches 3 and 3', remove them from the balance 90, while the pump 5 continues to draw the anticoagulant solution from the additional pouch 7. That the pump 5 operates in an open loop and receives no feedback from the balance 90 allows the operator to replace the main pouches 3 and 3' without being interrupted by signals or alarms caused by abnormal weight readings by the balance 90.

The full main pouches 3 and 3' are hung from the balance 90 and connected to the additional pouch 7, which resumes filling by gravity, and the treatment of the patient can continue without having experienced any interruption.

Advantageously, it is possible to provide clips 76 for closing the hydraulic ducts 70, 70' and 72 if necessary.

The operation of the apparatus for infusion of an anticoagulant solution is clear and evident from what has been described.

The pump 5 draws the anticoagulant solution from the additional pouch 7, by means of the duct 72, in order to introduce it in the blood circuit of the dialysis system, while the anticoagulant solution present in the main pouches 3 and 3' continues to fill by gravity the additional pouch 7 by means of the ducts 70 and 70'.

According to the invention, the method for replacing at least one main pouch 3, 3' in an infusion apparatus 1 as described above entails that when the main pouch 3, 3' is removed in order to be replaced, the pump 5 is kept running in order to continue drawing the anticoagulant solution from the additional pouch 7, therefore avoiding the interruption of the administration of anticoagulants to the patient and also preventing the intake of air by the pump 5. The pump 5 always draws the anticoagulant solution by means of the end 78 of the duct 72 that is always immersed in the anticoagulant solution that is present in the additional pouch 7.

In particular, the method for replacing the main pouch 3, 3' entails that the means for signaling the emptying of the main pouch 3, 3' are activated when the means 9 for detecting total weight detect a weight of anticoagulant solution that is substantially equal to a reference weight that corresponds to the weight of an additional pouch 7 filled with anticoagulant solution.

The means for signaling the emptying of the main pouch 3, 3' are activated when the control system detects a weight of anticoagulant solution that corresponds to the weight of anticoagulant solution present in a full additional pouch 7. From the time main pouch 3, 3' is empty and must be replaced, pump 5 can continue to run since it can draw the anticoagulant solution from the additional pouch 7.

For the removal of the main pouches 3 and 3' it is preferable to ensure that the ducts 70 and 70' have been closed beforehand by means of the clips 76.

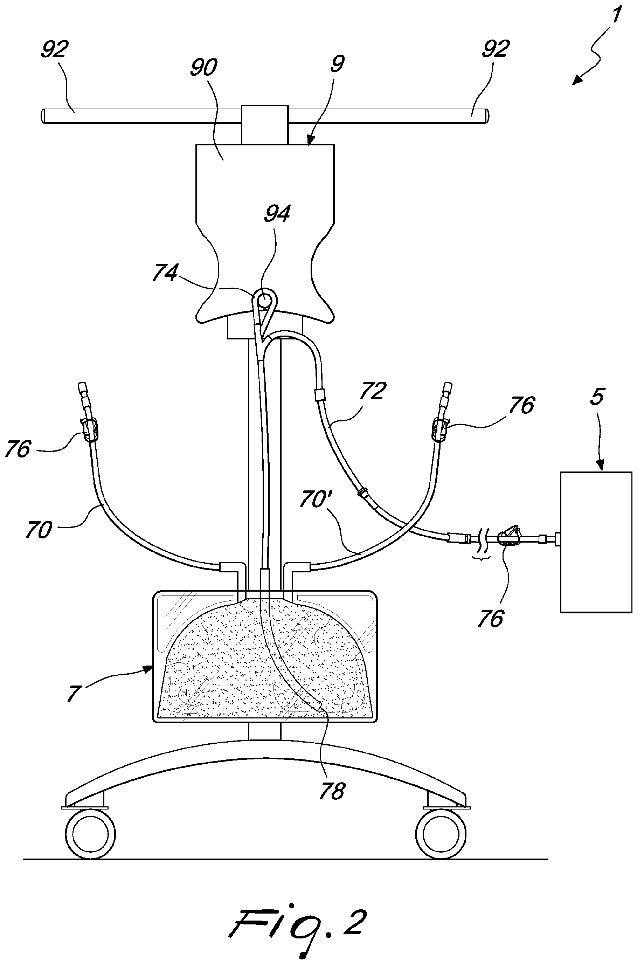

Figure 2 shows the infusion apparatus 1 when the main pouches 3 and 3' have been removed. The first ducts 70 and 70', detached from the corresponding main pouches 3 and 3', are preferably closed by means of the clips 76. The pump 5 is still running and draws the anticoagulant solution from the bottom of the additional pouch 7.

Advantageously, when the means 9 for detecting the total weight of the anticoagulant solution detect a weight of anticoagulant solution that is substantially equal to a reference weight that corresponds to the weight of an additional pouch 7 filled with anticoagulant solution, the pump 5 is kept running regardless of the information detected by the means 9 for detecting the total weight of the anticoagulant solution. In other words, in the step for replacement of the main pouches 3, 3', the control system operates in an open loop, ignoring the information that arrives from the balance 90 and drawing continuously the anticoagulant solution from the additional pouch 7.

Optionally, the additional pouch 7 of the solution infusion apparatus 1, which is configured to contain a solution to be introduced in a blood circuit of a dialysis system, comprises:
at least one first hydraulic duct 70, 70' configured to establish the hydraulic connection to the at least one main pouch 3, 3' of the solution infusion apparatus 1, and
a second hydraulic duct 72 configured to establish a hydraulic connection to the pump 5 of the infusion apparatus 1 to draw the solution from the additional pouch 7 and introduce it in the blood circuit of a dialysis system.

In practice it has been found that the solution infusion apparatus, or dialysis systems, system, as well as the corresponding method for replacing pouches containing said solution, according to the present invention, achieve the intended aim and objects, since they allow to ensure a continuous infusion of solution in the blood circuit, also preventing the unwanted introduction of air in the circuit.

Another advantage of the infusion apparatus, according to the invention, resides because it prevents human errors by the operator from compromising the correct administration of solution to the patient.

Another advantage of the infusion apparatus according to the invention resides in that it is simple and straightforward to use and in particular allows rapid pouch replacement.

Another advantage of the infusion apparatus according to the invention resides in that it allows complete emptying of the pouches prior to their replacement.

A further advantage of the infusion apparatus according to the invention resides in that the pump draws the solution always from the additional pouch and never from the main pouches and therefore, even in case of operator errors, the unwanted intake of air is impossible.

In the case of infusion of anticoagulant solutions, the advantage of eliminating the risk of coagulation is added to all of the advantages cited above.

In practice, the materials used, so long as they are compatible with the specific use, as well as the contingent shapes and dimensions, may be any according to requirements.

Where technical features mentioned in any claim are followed by reference signs, those reference signs have been included to increase the clarity of the claims and accordingly such reference signs have no limiting effect on the interpretation of each element identified by way of example by such reference signs.

## Claims

1. A solution infusion apparatus (1), for dialysis systems, comprising at least one main pouch (3, 3') adapted to contain a solution and comprising a pump (5) configured for introducing said solution in the blood circuit of a dialysis system; at least one additional pouch (7) that is hydraulically connected to said at least one main pouch (3, 3'), said pump (5) being configured to draw said solution from said at least one additional pouch (7);
and means (9) for detecting the total weight of said solution contained in said additional pouch (7) and in said at least one main pouch (3, 3');
**characterized in that** said additional pouch (7) is arranged at a lower level than said at least one main pouch (3, 3') so that said solution contained in said at least one main pouch (3, 3') fills by gravity said additional pouch (7),
and further comprises means for signaling the emptying of said at least one main pouch (3, 3') which are associated with said means (9) for detecting the total weight of said solution,
wherein if the means (9) for detecting the total weight detects a weight that corresponds to the weight of the solution present in the at least one additional pouch (7) alone, the apparatus is configured to signal this situation to the operator but keep the pump (5) running.

2. The solution infusion apparatus (1) according to claim 1, **characterized in that** said solution is an anticoagulant solution.

3. The solution infusion apparatus (1) according to one or more of the preceding claims, **characterized in that** it comprises a plurality of main pouches (3, 3'), each one of said main pouches (3, 3') being hydraulically connected to said at least one additional pouch (7).

4. The solution infusion apparatus (1) according to one or more of the preceding claims, **characterized in that** in the absence of solution in said at least one main pouch (3, 3') or in the absence of said at least one main pouch (3, 3'), said pump (5) is configured to perform in an open circuit said drawing of said solution from said at least one additional pouch (7).

5. The solution infusion apparatus (1) according to one or more of the preceding claims, wherein the at least one additional pouch (7) further comprises:
- at least one first hydraulic connection (70, 70') configured to establish a hydraulic connection to the at least one main pouch (3, 3') of the solution infusion apparatus (1), and
- a second hydraulic connection (72) configured to establish a hydraulic connection to the pump (5) of the infusion apparatus (1).

6. A method for replacing at least one main pouch (3, 3') in an infusion apparatus (1) according to one or more of claims 1 to 5, **characterized in that** when said at least one main pouch (3, 3') is removed to be replaced, said pump (5) is kept running to continue to draw said solution from the additional pouch (7).

7. The method according to claim 6, **characterized in that** during said replacement of said main pouch (3, 3'), said pump (5) is configured to operate in an open circuit so as to ignore the information that arrives from said means (9) for detecting the total weight of said solution and so as to draw continuously said solution from said at least one additional pouch (7).

8. The method according to claim 6 or 7, **characterized in that** said means for signaling the emptying of said at least one main pouch (3, 3') are activated when said means (9) for detecting the total weight of said solution detect a weight of the solution substantially equal to a reference weight that substantially corresponds to the weight of said additional pouch (7) full of said solution.

9. The method according to claim 6, 7 or 8, **characterized in that** when said means (9) for detecting the total weight of said solution detect a solution weight substantially equal to a reference weight that substantially corresponds to the weight of said additional pouch (7) full of said solution, said pump (5) is kept running independently of the information detected by said means (9) for detecting the total weight.

## Patentansprüche

1. Infusionseinrichtung (1) für eine Lösung für Dialysesysteme, die wenigstens einen Hauptbeutel (3, 3') umfasst, der angepasst ist, um eine Lösung zu enthalten, und eine Pumpe (5) umfasst, die zum Zuführen der Lösung in den Blutkreislauf eines Dialysesystems konfiguriert ist;
wenigstens einen zusätzlichen Beutel (7), der mit dem wenigstens einen Hauptbeutel (3, 3') hydraulisch verbunden ist, wobei die Pumpe (5) konfiguriert ist, um die Lösung aus dem wenigstens einen zusätzlichen Beutel (7) zu entnehmen; und
Mittel (9) zum Erfassen des Gesamtgewichts der Lösung, die in dem zusätzlichen Beutel (7) und in dem wenigstens einen Hauptbeutel (3, 3') enthalten ist;
**dadurch gekennzeichnet, dass** der zusätzliche Beutel (7) auf einer niedrigeren Höhe als der wenigstens eine Hauptbeutel (3, 3') angeordnet ist, so dass die in dem wenigstens einen Hauptbeutel (3, 3') enthaltene Lösung durch die Schwerkraft den zusätzlichen Beutel (7) füllt, und ferner Mittel zum Signalisieren des Entleerens des wenigstens einen Hauptbeutels (3, 3') umfasst, die mit dem Mittel (9) zum Erfassen des Gesamtgewichts der Lösung verknüpft sind, wobei, falls das Mittel (9) zum Erfassen des Gesamtgewichts ein Gewicht erfasst, das dem Gewicht der Lösung entspricht, die in dem wenigstens einen zusätzlichen Beutel (7) allein vorhanden ist, die Einrichtung konfiguriert ist, um dem Betreiber diese Situation zu signalisieren, aber die Pumpe (5) laufen zu lassen.

2. Infusionseinrichtung (1) für die Lösung nach Anspruch 1, **dadurch gekennzeichnet, dass** die Lösung eine gerinnungshemmende Lösung ist.

3. Infusionseinrichtung (1) für die Lösung nach einem oder mehreren der vorhergehenden Ansprüche, **dadurch gekennzeichnet, dass** sie mehrere Hauptbeutel (3, 3') umfasst, wobei jeder der Hauptbeutel (3, 3') mit dem wenigstens einen zusätzlichen Beutel (7) hydraulisch verbunden ist.

4. Infusionseinrichtung (1) für die Lösung nach einem oder mehreren der vorhergehenden Ansprüche, **dadurch gekennzeichnet, dass** in Abwesenheit der Lösung in dem wenigstens einen Hauptbeutel (3, 3') oder in Abwesenheit des wenigstens einen Hauptbeutels (3, 3') die Pumpe (5) konfiguriert ist, um in einem offenen Kreislauf die Entnahme der Lösung aus dem wenigstens einen zusätzlichen Beutel (7) durchzuführen.

5. Infusionseinrichtung (1) für die Lösung nach einem oder mehreren der vorhergehenden Ansprüche, wobei der wenigstens eine zusätzliche Beutel (7) ferner Folgendes umfasst:
- wenigstens eine erste hydraulische Verbindung (70, 70'), die konfiguriert ist, um eine hydraulische Verbindung mit dem wenigstens einen Hauptbeutel (3, 3') der Infusionseinrichtung (1) für die Lösung herzustellen, und
- eine zweite hydraulische Verbindung (72), die konfiguriert ist, um eine hydraulische Verbindung mit der Pumpe (5) der Infusionseinrichtung (1) herzustellen.

6. Verfahren zum Ersetzen wenigstens eines Hauptbeutels (3, 3') in einer Infusionseinrichtung (1) nach einem oder mehreren der Ansprüche 1 bis 5, **dadurch gekennzeichnet, dass**, wenn der wenigstens eine Hauptbeutel (3, 3') entfernt ist, um ersetzt zu werden, die Pumpe (5) laufen gelassen wird, um fortzufahren, die Lösung aus dem zusätzlichen Beutel (7) zu entnehmen.

7. Verfahren nach Anspruch 6, **dadurch gekennzeichnet, dass** während der Ersetzung des Hauptbeutels (3, 3') die Pumpe (5) konfiguriert ist, um in einem offenen Kreislauf in Betrieb zu sein, um die Informationen zu ignorieren, die von dem Mittel (9) zum Erfassen des Gesamtgewichts der Lösung ankommen und um die Lösung fortlaufend aus dem wenigstens einen zusätzlichen Beutel (7) zu entnehmen.

8. Verfahren nach Anspruch 6 oder 7, **dadurch gekennzeichnet, dass** die Mittel zum Signalisieren des Entleerens des wenigstens einen Hauptbeutels (3, 3') aktiviert sind, wenn das Mittel (9) zum Erfassen des Gesamtgewichts der Lösung ein Gewicht der Lösung erfasst, das im Wesentlichen gleich einem Referenzgewicht ist, das im Wesentlichen dem Gewicht des zusätzlichen Beutels (7) entspricht, der voll von der Lösung ist.

9. Verfahren nach Anspruch 6, 7 oder 8, **dadurch gekennzeichnet, dass**, wenn das Mittel (9) zum Erfassen des Gesamtgewichts der Lösung ein Lösungsgewicht erfasst, das im Wesentlichen gleich einem Referenzgewicht ist, das im Wesentlichen dem Gewicht des zusätzlichen Beutels (7) entspricht, der voll von der Lösung ist, die Pumpe (5) unabhängig von den Informationen, die durch das Mittel (9) zum Erfassen des Gesamtgewichts erfasst wurden, laufen gelassen wird.

## Revendications

1. Appareil de perfusion de solution (1), pour systèmes de dialyse, comprenant au moins une poche principale (3, 3') adaptée pour contenir une solution et comprenant une pompe (5) configurée pour introduire ladite solution dans le circuit sanguin d'un système de dialyse ;
au moins une poche supplémentaire (7) qui est reliée hydrauliquement à ladite au moins une poche principale (3, 3'), ladite pompe (5) étant configurée pour aspirer ladite solution de ladite au moins une poche supplémentaire (7) ; et
des moyens (9) pour détecter le poids total de ladite solution contenue dans ladite poche supplémentaire (7) et dans ladite au moins une poche principale (3, 3') ;
**caractérisé en ce que** ladite poche supplémentaire (7) est agencée à un niveau inférieur à ladite au moins une poche principale (3, 3') de sorte que ladite solution contenue dans ladite au moins une poche principale (3, 3') remplit par gravité ladite poche supplémentaire (7), et comprend en outre des moyens pour signaler la vidange de ladite au moins une poche principale (3, 3') qui sont associés auxdits moyens (9) pour détecter le poids total de ladite solution,
dans lequel si les moyens (9) pour détecter le poids total détectent un poids qui correspond au poids de la solution présente dans l'au moins une poche supplémentaire (7) seule, l'appareil est configuré pour signaler cette situation à l'opérateur tout en maintenat la pompe (5) en marche.

2. Appareil de perfusion de solution (1) selon la revendication 1, **caractérisé en ce que** ladite solution est une solution anticoagulante.

3. Appareil de perfusion de solution (1) selon une ou plusieurs des revendications précédentes, **caractérisé en ce qu'**il comprend une pluralité de poches principales (3, 3'), chacune desdites poches principales (3, 3') étant reliée hydrauliquement à ladite au moins une poche supplémentaire (7).

4. Appareil de perfusion de solution (1) selon une ou plusieurs des revendications précédentes, **caractérisé en ce qu'**en l'absence de solution dans ladite au moins une poche principale (3, 3') ou en l'absence de ladite au moins une poche principale (3, 3'), ladite pompe (5) est configurée pour effectuer en circuit ouvert ladite aspiration de ladite solution à partir de ladite au moins une poche supplémentaire (7).

5. Appareil de perfusion de solution (1) selon une ou plusieurs des revendications précédentes, dans lequel l'au moins une poche supplémentaire (7) comprend en outre :
- au moins une première liaison hydraulique (70, 70') conçue pour établir une liaison hydraulique avec l'au moins une poche principale (3, 3') de l'appareil de perfusion de solution (1), et
- une seconde liaison hydraulique (72) conçue pour établir une liaison hydraulique avec la pompe (5) de l'appareil de perfusion (1).

6. Procédé pour remplacer au moins une poche principale (3, 3') dans un appareil de perfusion (1) selon une ou plusieurs des revendications 1 à 5, **caractérisé en ce que** lorsque ladite au moins une poche principale (3, 3') est retirée pour être remplacée, ladite pompe (5) est maintenue en marche pour continuer à aspirer ladite solution de la poche supplémentaire (7).

7. Procédé selon la revendication 6, **caractérisé en ce que** lors dudit remplacement de ladite poche principale (3, 3'), ladite pompe (5) est configurée pour fonctionner en circuit ouvert de manière à ignorer les informations qui arrivent desdits moyens (9) pour détecter le poids total de ladite solution et de manière à aspirer en continu ladite solution de ladite au moins une poche supplémentaire (7).

8. Procédé selon la revendication 6 ou 7, **caractérisé en ce que** lesdits moyens pour signaler la vidange de ladite au moins une poche principale (3, 3') sont activés lorsque lesdits moyens (9) pour détecter le poids total de ladite solution détectent un poids de la solution sensiblement égal à un poids de référence qui correspond sensiblement au poids de ladite poche supplémentaire (7) pleine de ladite solution.

9. Procédé selon la revendication 6, 7 ou 8, **caractérisé en ce que** lorsque lesdits moyens (9) pour détecter le poids total de ladite solution détectent un poids de solution sensiblement égal à un poids de référence qui correspond sensiblement au poids de ladite poche supplémentaire (7) pleine de ladite solution, ladite pompe (5) est maintenue en marche indépendamment des informations détectées par lesdits moyens (9) de détection du poids total.
